# EUROPEAN PATENT APPLICATION

(11) **EP 2 901 926 A1**
(43) Date of publication of application: **05.08.2015**
(21) Application number: 15153022.7
(22) Date of filing: 29.01.2015
(51) Int. Cl.: A61B 5/145, A61B 5/00

(54) **Blood glucose level measurement apparatus and blood glucose level measurement method**

(30) Priority: 31.01.2014 JP 2014017023
(71) Applicant: Seiko Epson Corporation, Tokyo 163 (JP)
(72) Inventor: Kasahara, Hirokazu, Nagano, 392-8502 (JP); Mizobe, Kimitake, Nagano, 392-8502 (JP); Ishiguro, Hideto, Nagano, 392-8502 (JP)
(74) Representative: Hoffmann Eitle

(57) **Abstract**

A blood glucose level measurement apparatus is a non-invasive measurement apparatus mounted on a wrist or the like of a user and using light, and intermittently measures a blood glucose level of the user. A measurement interval is set according to a measured blood glucose level. For example, in a case where the measured blood glucose level is low, the measurement interval is set to be short.

## Description

### BACKGROUND

### 1. Technical Field

The present invention relates to a blood glucose level measurement apparatus and the like which measure a blood glucose level of a user.

### 2. Related Art

In recent years, as a portable blood glucose level measurement apparatus which allows a diabetic to measure a blood glucose level himself/herself, development of a non-invasive blood glucose level measurement apparatus using light has progressed. A blood glucose level greatly varies in a day depending on a meal or exercise and is thus required to be frequently measured. Therefore, a portable blood glucose level measurement apparatus is desirable on the condition of its continuous use. However, the apparatus is portable and is thus required to operate by using a battery. From the viewpoint of the use time, or a frequency of charging or exchanging a battery, a large-capacity battery is desirable, but from the viewpoint of portability, a small-capacity battery which is small and light weight is desirable. In either viewpoint, reducing power consumption is an important technique.

As a technique for reducing power consumption, for example, JP-A-10-314150 discloses a technique in which measurement is intermittently performed in an apparatus which measures a blood component.

Meanwhile, first of all, the continuous measurement of a blood glucose level is aimed at rapidly detecting a low blood glucose state in which a blood glucose level is low. In a case of employing the technique in which the measurement is intermittently performed as disclosed in JP-A-10-314150, if a measurement interval is set to be too long, there is a risk that detection of the low blood glucose state may be late. The low blood glucose state is life-threatening and is thus required to be rapidly detected. For this reason, how to set a measurement interval is important.

### SUMMARY

An advantage of some aspects of the invention is to provide a novel technique of enabling a low blood glucose state to be rapidly detected and power consumption to be reduced in a portable blood glucose level measurement apparatus.

A first aspect of the invention is directed to a blood glucose level measurement apparatus including a measurement unit that measures a blood glucose level of a user; and a measurement interval setting unit that sets a measurement interval performed by the measurement unit on the basis of a measurement result from the measurement unit.

As another aspect of the invention, the first aspect may be configured as a blood glucose level measurement method including performing a measurement of a blood glucose level of a user; and setting an interval of the measurement on the basis of a result of the measurement.

According to the first aspect and the like, it is possible to set a measurement interval on the basis of a measurement result of a blood glucose level of the user. In other words, since a measurement interval can be dynamically changed according to a blood glucose level, for example, in a case where it is determined that a blood glucose level approaches a low blood glucose state, a measurement interval can be shortened. Thus, it is possible to rapidly detect the low blood glucose state and to reduce power consumption.

As a second aspect of the invention, the blood glucose level measurement apparatus according to the first aspect may be configured such that the measurement interval setting unit sets a first measurement interval in a case where the measurement result is in a first range, and sets a second measurement interval shorter than the first measurement interval in a case where the measurement result is in a second range shorter than the first range.

According to the second aspect, the first measurement interval is set in a case where the measurement result is in the first range, and the second measurement interval shorter than the first measurement interval is set in the case where the measurement result is in the second range shorter than the first range. In other words, a measurement interval is changed and set in at least two stages depending on a blood glucose level. Consequently, in a case where a measured blood glucose level is low, a measurement interval is set to be short, that is, a measured frequency is increased, and thus a low blood glucose state can be rapidly detected.

As a third aspect of the invention, the blood glucose level measurement apparatus according to the first or second aspect may be configured such that the blood glucose level measurement apparatus further includes a storage unit that stores the measurement result, and the measurement interval setting unit includes a prediction portion that predicts a blood glucose level by using a measurement result stored in the storage unit, and sets the measurement interval by using a prediction result.

According to the third aspect, a measurement result is stored, a blood glucose level is predicted by using the stored measurement result, and a measurement interval is set by using the prediction result. Therefore, for example, a future blood glucose level or a future change in a blood glucose level can be predicted on the basis of a time-series change in a measurement result, and thus it is possible to improve accuracy of blood glucose level prediction. As a result, it is possible to set a more appropriate measurement interval.

As a fourth aspect of the invention, the blood glucose level measurement apparatus according to the third aspect may be configured such that the measurement interval setting unit includes a determination portion that determines a point of time at which the prediction result satisfies a predetermined condition, and sets the measurement interval so that the measurement unit performs the next measurement at the point of time.

According to the fourth aspect, a point of time at which the prediction result satisfies a predetermined condition is determined, and the measurement interval is set so that the next measurement is performed at the point of time. The predetermined condition is set to, for example, a condition in which it can be determined that a blood glucose level is a low blood glucose state equal to or lower than a predetermined value, and thus a blood glucose level can be measured before a point of time at which the low blood glucose state is predicted to occur. Therefore, it is possible to rapidly detect the low blood glucose state or whether or not a blood glucose level approaches the low blood glucose state.

As a fifth aspect of the invention, the blood glucose level measurement apparatus according to the third or fourth aspect may be configured such that the blood glucose level measurement apparatus includes an acquisition unit that acquires a scheduled action of the user, and the measurement interval setting unit includes a prediction result adjustment portion that adjusts the prediction result on the basis of the scheduled action.

According to the fifth aspect, the prediction result of the blood glucose level is adjusted on the basis of the scheduled action of the user. Consequently, in consideration of an external factor which causes a blood glucose level to change, a blood glucose level is predicted with higher accuracy, and a more appropriate measurement interval can be set.

As a sixth aspect of the invention, the blood glucose level measurement apparatus according to the fifth aspect may be configured such that the prediction result adjustment portion adjusts and reduce a predicted value of a blood glucose level included in the prediction result in a case where the scheduled action is an action related to exercise or insulin administration.

According to the sixth aspect, a predicted value of a blood glucose level is adjusted to be reduced in a case where the scheduled action of the user is an action related to exercise or insulin administration. This is because a blood glucose level generally decreases due to exercise or insulin administration.

As a seventh aspect of the invention, the blood glucose level measurement apparatus according to the fifth or sixth aspect may be configured such that the prediction result adjustment portion adjusts and increase a predicted value of a blood glucose level included in the prediction result in a case where the scheduled action is an action related to a meal.

According to the seventh aspect, a predicted value of a blood glucose level is adjusted to be increased in a case where the scheduled action is an action related to a meal. This is because a blood glucose level generally increases due to a meal.

### BRIEF DESCRIPTION OF THE DRAWINGS

The invention will now be described with reference to the accompanying drawings, wherein like numbers reference like elements.
Fig. 1 is an exterior example of a blood glucose level measurement apparatus.
Figs. 2A and 2B are configuration diagrams of a sensor module.
Fig. 3 is a diagram for explaining acquisition of a biological image.
Fig. 4 is a diagram illustrating an example of a biological image.
Fig. 5 is a diagram for explaining acquisition of a blood vessel pattern.
Fig. 6 is a diagram for explaining selection of a measurement target blood vessel part.
Fig. 7 is a diagram for explaining selection of an irradiation position and a light reception position.
Fig. 8 is a diagram for explaining the optimum distance between the irradiation position and the light reception position.
Fig. 9 is a diagram for explaining a light emission range.
Fig. 10 is a diagram for explaining an imaging range based on the light emission range.
Fig. 11 is a diagram illustrating a functional configuration of a blood glucose level measurement apparatus in a first example.
Fig. 12 is a diagram illustrating a configuration example of blood vessel part data.
Fig. 13 is a diagram illustrating a data configuration example of a measurement interval setting table.
Fig. 14 is a flowchart illustrating a blood glucose level measurement process in the first example.
Fig. 15 is a diagram for explaining prediction of a blood glucose level.
Figs. 16A and 16B are diagrams for explaining adjustment of a prediction result of a blood glucose level based on an action.
Fig. 17 is a diagram for explaining determination of a low glucose state based on the prediction result of a blood glucose level.
Fig. 18 is a diagram illustrating a functional configuration of a blood glucose level measurement apparatus in a second example.
Fig. 19 is a diagram illustrating a configuration example of scheduled action data.
Fig. 20 is a flowchart illustrating a blood glucose level measurement process in the second example.
Fig. 21 is a diagram illustrating a data configuration example of a blood glucose level adjustment amount table.

### DESCRIPTION OF EXEMPLARY EMBODIMENTS

### Entire Configuration

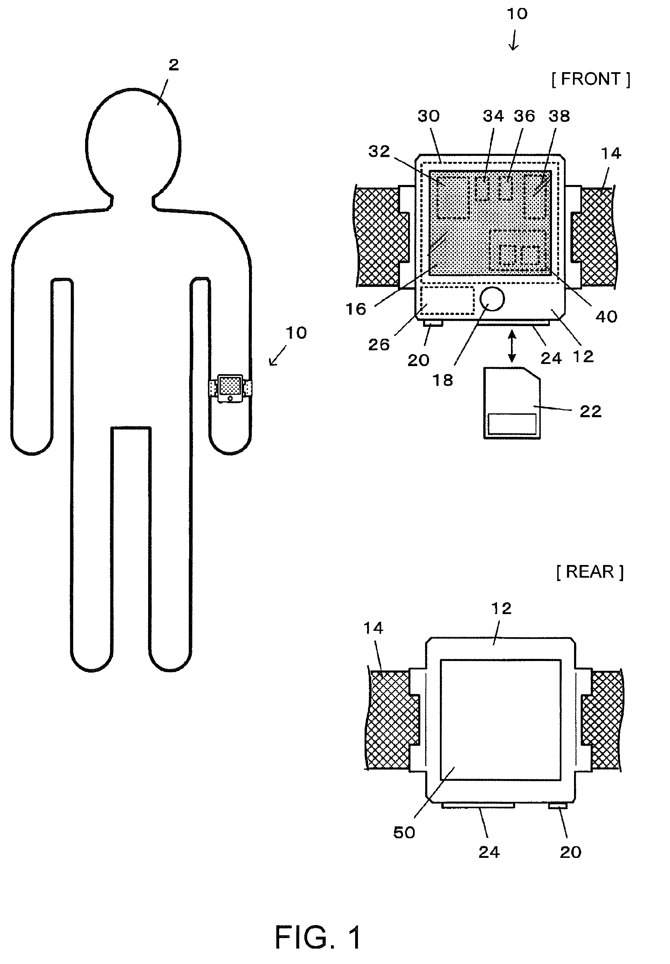

Fig. 1 is a diagram illustrating a configuration example of a blood glucose level measurement apparatus 10 of the present embodiment. The blood glucose level measurement apparatus 10 measures a blood glucose level which is a glucose concentration in blood of a user 2 by using light in a non-invasive manner. As illustrated in Fig. 1, the blood glucose level measurement apparatus 10 is of a wearable apparatus which is a wrist watch type and includes a main body case 12, and a fixation band 14 such as Velcro tape (registered trademark) for mounting and fixing the main body case 12 on and to a measurement part such as the wrist or the arm of the user 2.

A touch panel 16 and an operation switch 18 are provided on a front surface (a surface directed outwards when the user 2 wears the blood glucose level measurement apparatus 10) of the main body case 12. The user 2 inputs a measurement start instruction by using the touch panel 16 or the operation switch 18, or a measurement result is displayed on the touch panel 16.

A communication device 20 for communication with external devices and a reader/writer 24 of a memory card 22 are provided on a side surface of the main body case 12. The communication device 20 is implemented by a jack for attaching and detaching a wired cable, or a wireless communication module and an antenna for performing wireless communication. The memory card 22 is a data rewritable nonvolatile memory such as a flash memory, a ferroelectric random access memory (FeRAM), or a magnetoresistive random access memory (MRAM).

A sensor module 50 is provided on a rear surface of the main body case 12 so as to be in contact with a skin surface of the user 2. The sensor module 50 is a measurement device which irradiates the skin surface of the user 2 with measurement light and receives reflected or transmitted light, and is a thin image sensor having a light source therein.

A charge type battery 26 and a control board 30 are built into the main body case 12. As a method of charging the battery 26, there may be a configuration in which an electric contact is provided on the rear surface side of the main body case 12, the main body case 12 is set in a cradle connected to a domestic power supply, and the battery 26 is charged via the electric contact and the cradle, or the battery 26 may be charged in a wireless manner.

The control board 30 is equipped with a central processing unit (CPU), a main memory, a measurement data memory, a touch panel controller, and a sensor module controller. The main memory is a storage medium which stores a program or initial setting data or stores calculation values of the CPU, and is implemented by a RAM, a read only memory (ROM), a flash memory, or the like. The program or the initial setting data may be stored in the memory card 22. The measurement data memory is a storage medium which stores measurement data, and is implemented by a data rewritable nonvolatile memory such as a flash memory, a ferroelectric random access memory (FeRAM), or a magnetoresistive random access memory (MRAM). The measurement data may be stored in the memory card 22.

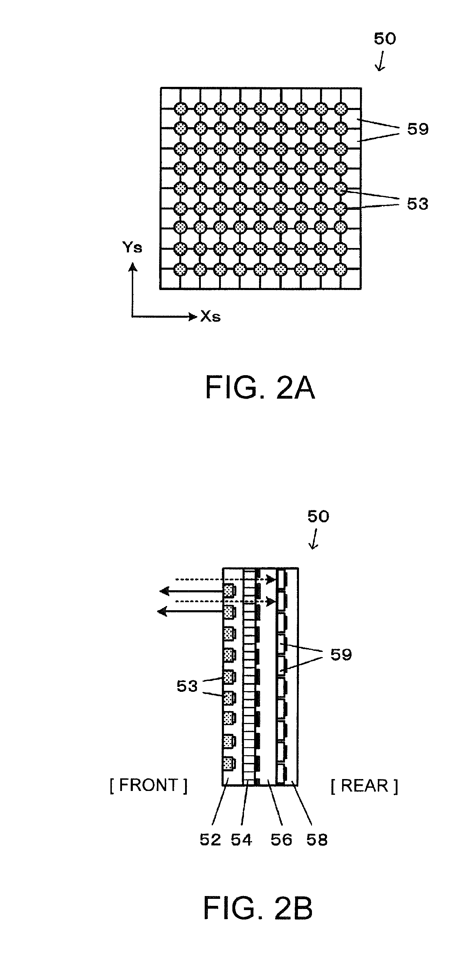

Figs. 2A and 2B are diagrams illustrating a configuration of the sensor module 50. Fig. 2A is a plan view and Fig. 2B is a cross-sectional view. The sensor module 50 is a device in which a light emitting layer 52, a light blocking layer 54, a spectroscopic layer 56, and a light receiving layer 58 are laminated. A plurality of light emitting elements 53 are arranged in a planar shape in a two-dimensional manner in the light emitting layer 52, and the light blocking layer 54 selectively blocks light which is not directed to the light receiving layer 58. The spectroscopic layer 56 selectively transmits near infrared rays therethrough, and a plurality of light receiving elements 59 are arranged in a planar shape in a two-dimensional manner in the light receiving layer 58. The sensor module 50 is provided on the rear side of the main body case 12 so that a front side thereof (a surface of the light emitting layer 52 side) is directed to the skin surface of the user 2.

The light emitting elements 53 are an irradiation unit which applies measurement light, and are implemented by, for example, light emitting diodes (LEDs), organic light emitting diodes (OLEDs), or the like. In the present embodiment, in order to measure a blood glucose level (a glucose concentration in blood), the light emitting elements 53 employ elements which can emit light including near infrared rays having subcutaneous transparency.

The light receiving elements 59 are a light receiving unit which receives transmitted light or reflected light of the measurement light and outputs an electric signal corresponding to a light reception amount, and are implemented by imaging elements such as charge coupled device (CCD) image sensors or complementary metal oxide semiconductor (CMOS) image sensors. A single light receiving element 59 includes a plurality of elements which receive wavelength components required for calibration.

The light emitting elements 53 of the light emitting layer 52 and the light receiving elements 59 of the light receiving layer 58 are disposed in a matrix which is defined in a common Xs-Ys orthogonal coordinate system. In addition, the light emitting elements 53 of the light emitting layer 52 and the light receiving elements 59 of the light receiving layer 58 are arranged at the same intervals in directions of the Xs and Ys axes but are alternately arranged in the Xs-Ys plane. In other words, positions of the light emitting elements 53 and the light receiving elements 59 in the directions of the Xs and Ys axes are laminated so as to be deviated relative to each other by a predetermined length. Consequently, light which has been transmitted through a biological tissue of the user 2 or light which has been reflected inside the biological tissue (hereinafter, the light is referred to as reflected/transmitted light as appropriate) can reach the light receiving elements 59.

An arrangement interval of each of the light emitting elements 53 in the light emitting layer 52 and the light receiving elements 59 in the light receiving layer 58 may be set as appropriate. For example, an arrangement interval of 1 to 500 [µm] is preferably used, and, for example, 50 to 200 [µm] may be used from the viewpoint of manufacturing cost and measurement accuracy. The light emitting elements 53 and the light receiving elements 59 are not only laminated but may also be arranged in parallel to each other.

### Measurement Principle

A description will be made of a measurement principle of a blood glucose level in the present embodiment. In order to perform a measurement, the blood glucose level measurement apparatus 10 is fixed with the fixation band 14 so that the sensor module 50 is brought into close contact with the skin surface of the user 2. Since the sensor module 50 is brought into close contact with the skin surface, it is possible to minimize factors which reduce measurement accuracy, such as reflection of measurement light at the skin surface or scattering thereof near the skin surface. A blood vessel in a biological tissue directly under the sensor module 50 is set as a measurement target, light including transmitted light which is a result of measurement light being transmitted through the blood vessel is received so as to obtain an absorption spectrum, and a blood glucose level is estimated and calculated.

### (A-1) Acquisition of blood vessel pattern

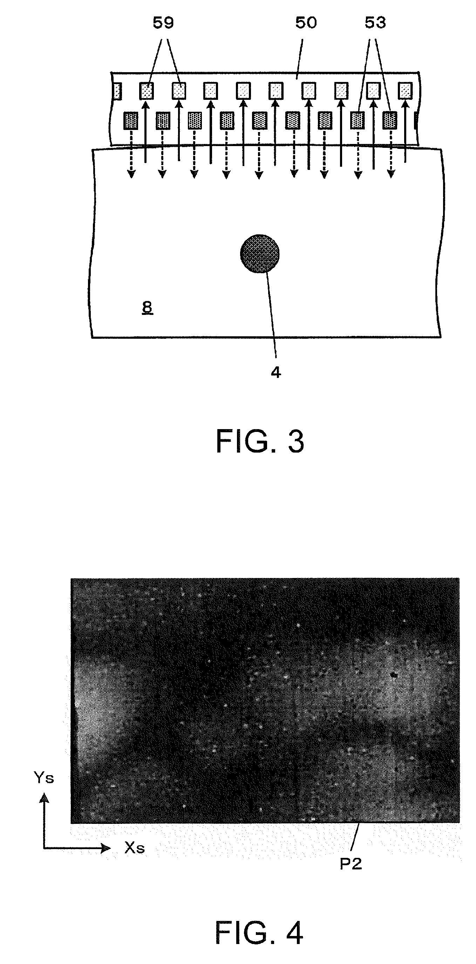

Specifically, first, a blood vessel pattern (a position of a blood vessel) which is viewed from the skin surface is acquired. The acquisition of a blood vessel pattern may be performed in the same manner as in the vein pattern detection of the well-known vein authentication technique. In other words, as schematically illustrated in Fig. 3, the light emitting elements 53 of the sensor module 50 are made to simultaneously emit light so that the skin surface of the user 2 is irradiated with measurement light. In addition, all of the light receiving elements 59 are used to receive light (transmitted light) which is a result of the measurement light being transmitted through the biological tissue or light (reflected light) which is a result of the measurement light being reflected in the biological tissue so as to capture an image of the biological tissue, thereby acquiring a biological image.

Fig. 4 is a diagram illustrating an example of a biological image P2. The biological image P2 can be obtained as a two-dimensional image having the same number of pixels as in the arrangement of the light receiving elements 59 of the sensor module 50.

Since the blood vessel more easily absorbs near infrared rays than a non-blood vessel portion, the blood vessel portion has lower luminance and is darker than the non-blood vessel portion in the biological image P2. For this reason, the portion having the low luminance in the biological image P2 is extracted, and thus a blood vessel pattern can be extracted. In other words, on the basis of whether or not the luminance of each pixel forming the biological image P2 is equal to or lower than a predetermined threshold value, the presence or absence of a blood vessel directly under the light receiving elements 59, that is, a position of the blood vessel, can be acquired.

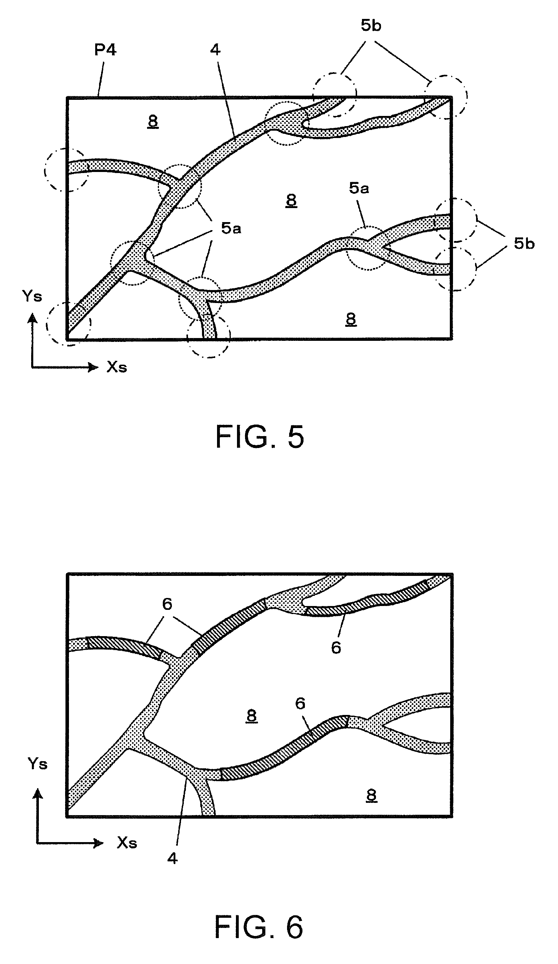

Fig. 5 is a diagram illustrating an example of a blood vessel pattern P4 obtained on the basis of the biological image P2 of Fig. 4. The blood vessel pattern P4 is information indicating whether a corresponding region is a blood vessel region or a non-blood vessel region for each pixel forming the biological image, that is, for each position of the light receiving element 59. In Fig. 5, a shaded strip-shaped portion is a blood vessel 4, and the other white portion is extracted as a non-blood vessel region 8.

### (A-2) Selection of measurement target blood vessel part

If the blood vessel pattern is acquired, then, a measurement target blood vessel (more specifically, a blood vessel part) is selected. A measurement target blood vessel part 6 is selected so as to satisfy the following selection condition. The selection condition is that "the blood vessel part is a part other than a branching portion or a joint portion of the blood vessel and an end portion of the image, and has a predetermined length and a predetermined width in a blood vessel length direction".

In branch or joint portions 5a (refer to Fig. 5) of the blood vessel, light which has passed through a blood vessel which is not a measurement target may be mixed with received light. The light which has passed through the blood vessel other than the measurement target blood vessel part 6 may influence an absorption spectrum of the measurement target blood vessel part 6 and thus measurement accuracy may decrease. For this reason, the measurement target blood vessel part 6 is selected from blood vessel portions excluding the branch or joint portions 5a of the blood vessel.

In end portions 5b (refer to Fig. 5) of the biological image, a structure such as branch or joint of the blood vessel near the outside of the image is unclear, and thus measurement accuracy may decrease for the above-described reason. In order to prevent this situation, the measurement target blood vessel part 6 is selected from blood vessel portions excluding the image end portions 5b.

In addition, irradiation light from the light emitting elements 53 is diffused and reflected inside a biological tissue, and some of the reflected light is received by the light receiving elements 59. In other words, some of the light received by the light receiving element 59 becomes transmitted light of the target blood vessel, but as a ratio of the transmitted light increases, an absorption spectrum which more remarkably represents a feature of a blood component of the target blood vessel may be obtained. In other words, measurement accuracy increases.

In addition, a relatively thinly reflected blood vessel (a short blood vessel in the width direction) is an originally thin blood vessel, and is a blood vessel which is located at a relatively deep position. An amount of transmitted light is small in this blood vessel, and thus measurement accuracy may decrease. For this reason, the measurement target blood vessel part 6 is selected from blood vessel portions (that is, blood vessel parts having a predetermined width) excluding the thinly reflected blood vessel.

Fig. 6 is a diagram illustrating an example of the measurement target blood vessel part 6 obtained on the basis of the blood vessel pattern P4 of Fig. 5. In Fig. 6, a hatched portion of the blood vessel 4 is the blood vessel part 6 which is selected as a measurement target.

### (A-3) Selection of irradiation position and light reception position

Next, in relation to the selected measurement target blood vessel part 6, an irradiation position (measurement light emitting element) of measurement light, an light reception position (measurement light receiving element) which is suitable for receiving transmitted light of the measurement target blood vessel part 6, and a light reception position (reference light receiving element) which is suitable for obtaining reference transmitted light, are selected. The reference transmitted light is light which is not transmitted through the measurement target blood vessel part 6 but is transmitted only through the non-blood vessel region 8 near the blood vessel part 6.

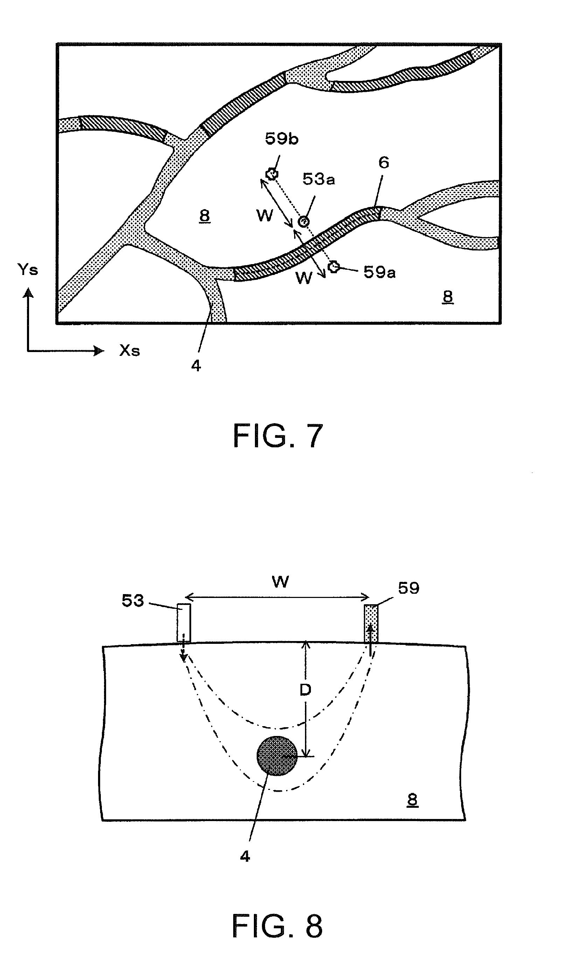

Fig. 7 is a diagram for explaining selection of the irradiation position, the measurement light reception position, and the reference light reception position. First, the irradiation position (measurement light emitting element) and the measurement light reception position (measurement light receiving element) are selected so as to satisfy the following first relative position condition. The first relative position condition is that, in the blood vessel pattern, "the measurement target blood vessel part 6 is located at a center between the irradiation position and the measurement light reception position and a distance between the irradiation position and the measurement light reception position is the same as a predetermined optimum distance W". The light emitting element 53 at the irradiation position satisfying the first relative position condition is set as a measurement light emitting element 53a, and the light receiving element 59 at the measurement light reception position is set as a measurement light receiving element 59a.

In addition, the irradiation position (measurement light emitting element) and the reference light reception position (reference light receiving element) are selected so as to satisfy the following second relative position condition. The second relative position condition is that, in the blood vessel pattern, "there is no blood vessel between the irradiation position and the reference light reception position and a distance between the irradiation position and the reference light reception position is the same as the predetermined optimum distance W". The light receiving element 59 at the reference light reception position satisfying the second relative position condition is set as a reference light receiving element 59b.

In the present embodiment, the reference light reception position is located on an extension of the line connecting the measurement irradiation position and the measurement light reception position to each other satisfying the above-described first relative position condition and is located opposite to the measurement light reception position when viewed from the measurement irradiation position, but is not limited thereto. It is assumed that none of the measurement irradiation position (the measurement light emitting element 53a), the measurement light reception position (the measurement light receiving element 59a), and the reference light reception position (reference light receiving element) are located over the blood vessel 4 (the positions are located in the non-blood vessel region 8).

The optimum distance W is defined as follows. Fig. 8 is a diagram for explaining propagation of light in a biological tissue, and is a cross-sectional view in a depth direction. Light applied from the light emitting element 53 is diffused and reflected inside the biological tissue, and some of the applied light reaches a certain light receiving element 59. A propagation path of the light forms a so-called banana shape (a region interposed between two arcs), a width in the depth direction is largest in the vicinity of approximately a center thereof, and the entire depth (reachable depth) is increased in accordance with a gap between the light emitting element 53 and the light receiving element 59.

If measurement accuracy is to be increased, it is desirable that an amount of transmitted light which is greater than an amount of light transmitted through the blood vessel 4 is received by the light receiving element 59. From this factor, the target blood vessel 4 is located nearly at a center between the light emitting element 53 and the light receiving element 59, and the optimum distance W is defined according to an expected depth D of the target blood vessel 4. The optimum distance W, that is, the optimum gap W between the light emitting element 53 and the light receiving element 59 is a distance which approximately doubles a depth D of the blood vessel 4 from the skin surface. For example, if the depth D is about 3 mm, the optimum distance W is about 5 mm to 6 mm.

### (A-4) Measurement

If the irradiation position and the light reception position are determined for the measurement target blood vessel part 6, a blood glucose level is measured. In other words, measurement light is applied from the measurement irradiation position (the measurement light emitting element 53a) set for the measurement target blood vessel part 6, and an absorption spectrum is generated on the basis of each light reception result at the measurement light reception position (the measurement light receiving element 59a) and the reference light reception position (the reference light receiving element 59b).

In this case, for example, a wavelength of light emitted by the light emitting element 53 is changed so that a wavelength λ of light applied to the skin surface is changed within a near infrared region, and thus the transmittance of the blood vessel part 6 is obtained for each wavelength λ. The transmittance T (λ) is obtained as T (λ) =Os (λ) /Or (λ) on the basis of light intensity Os(λ) obtained by the measurement light receiving element 59a and light intensity Or(λ) obtained by the reference light receiving element 59b. An absorption ratio is obtained on the basis of the transmittance, and then an absorption spectrum is generated.

Here, a calculation principle of the transmittance R will be described briefly. Generally, when the intensity of light applied by the light emitting element 53 is P(λ), the transmittance of an object portion through which the irradiation light is transmitted is T(λ), and the sensitivity defined for the light receiving element 59 is S(λ), a light intensity O(λ) obtained by the light receiving element 59 is given as O(λ)=P(λ)·T(λ)·S(λ).

From this relational expression, the light intensity Or(λ) obtained by the reference light receiving element 59b, not including transmitted light of the blood vessel 4, becomes Or (λ) =P (λ) ·T (λ) -S (λ) assuming that the transmittance T (λ) of the non-blood vessel region portion is "1".

In addition, the light intensity Os(λ) obtained by the measurement light receiving element 59a, including transmitted light of the blood vessel 4, becomes Os (λ) =P (λ) ·T (λ) ·S(λ). From the two equations, the transmittance T(λ) is obtained. The transmittance T(λ) is a relative value for the transmittance of the non-blood vessel region 8.

### (A-5) Calculation of blood glucose level

Next, on the basis of the absorption spectrum, a blood glucose level is estimated and calculated by using a calibration curve indicating a relationship between a predefined blood glucose level (a glucose concentration in blood) and an absorbance. In addition, a technique for calculating a concentration of a predetermined component (glucose in the present embodiment) on the basis of the absorption spectrum is well known, and the well-known technique may be employed in the present embodiment.

Two Examples applied to the blood glucose level measurement apparatus 10 having the above-described configuration will be described in order.

### First Example

### Overview

In the first example, a blood glucose level is intermittently measured so that power saving is realized and a measurement interval is varied depending on a measurement result. Specifically, in order to rapidly detect a reduction in a blood glucose level, as a measured blood glucose level becomes lower, a measurement interval to the next measurement is set to become shorter. In the continuous measurement of a blood glucose level (that is, a blood glucose level can be said to be monitored), it is important to rapidly detect a reduction in a blood glucose level (low blood glucose). For this reason, in a case where a blood glucose level is in a normal range, and thus a risk of low blood glucose is low, a measurement interval is set to be long so that power saving is prioritized. On the other hand, in a case where a blood glucose level is low and thus a risk of low blood glucose is high, a measurement interval is set to be short so that a reduction in a blood glucose level is rapidly detected.

In the present embodiment, when a blood glucose level is measured and a biological image is acquired, not all of the light emitting elements 53 of the sensor module 50 emit light but only some of the light emitting elements 53 emit light, and thus power saving is further realized. The light emitting elements 53 which are made to emit light during the acquisition of a biological image are determined on the basis of an irradiation position (the measurement light emitting element 53a) in the previous measurement.

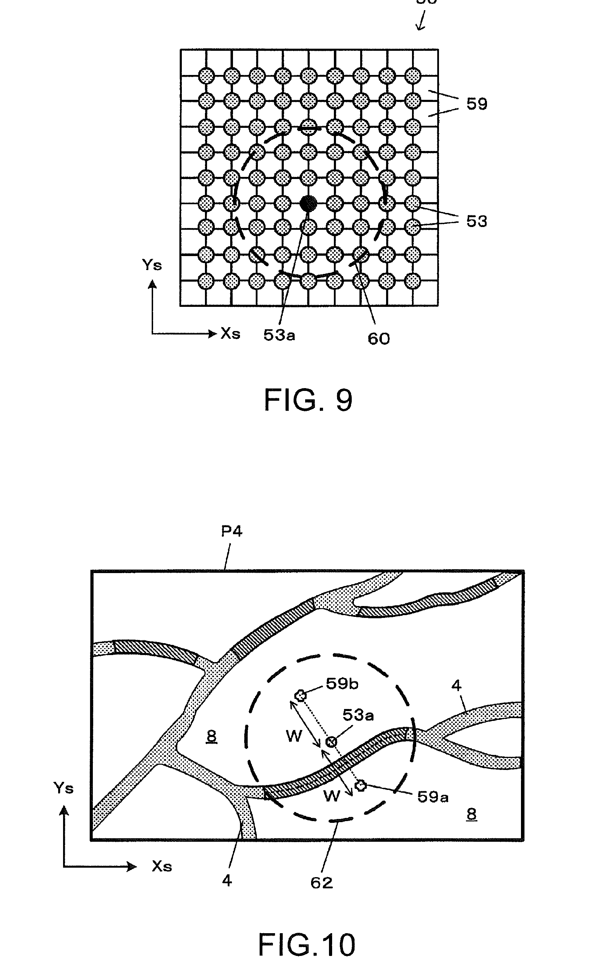

Fig. 9 is a diagram for explaining selection of the light emitting element 53 which is made to emit light during the acquisition of a biological image, and is a plan view of the sensor module 50. As illustrated in Fig. 9, a light emission range 60 which is a circular range with a predetermined radius R is defined centering on the measurement light emitting element 53a in the previous measurement, and only the light emitting elements 53 in this light emission range 60 are made to emit light.

The radius R may be set to be longer than the optimum distance W which is used to set, for example, the measurement light reception position and the reference light reception position. Since the blood glucose level measurement apparatus 10 of the present embodiment is mounted on the wrist or the like of the user 2 and is used, there is a high possibility that a relative positional relationship between the sensor module 50 and the skin surface of the user 2 may not be changed much from the previous measurement. For this reason, in a case where the light emission range 60 is defined as described above, there is a high possibility that the measurement target blood vessel part 6 in the previous measurement may be included in a range 62 (imaging range) of an acquired biological image as illustrated in Fig. 10.

Since the radius R is defined to be longer than the optimum distance W, the imaging range 62 includes the measurement target blood vessel part 6 (specifically, the blood vessel part 6 interposed between the irradiation position (the measurement light emitting element 53a) and the measurement light reception position (the measurement light receiving element 59a)) in the previous measurement.

In a case where a mounting position of the blood glucose level measurement apparatus 10 is deviated due to remounting or the like, the blood vessel 4 may not be included in an acquired biological image. In this case, the radius R is reset to be long so as to widen the light emission range 60, and a biological image may be acquired again.

### Functional Configuration

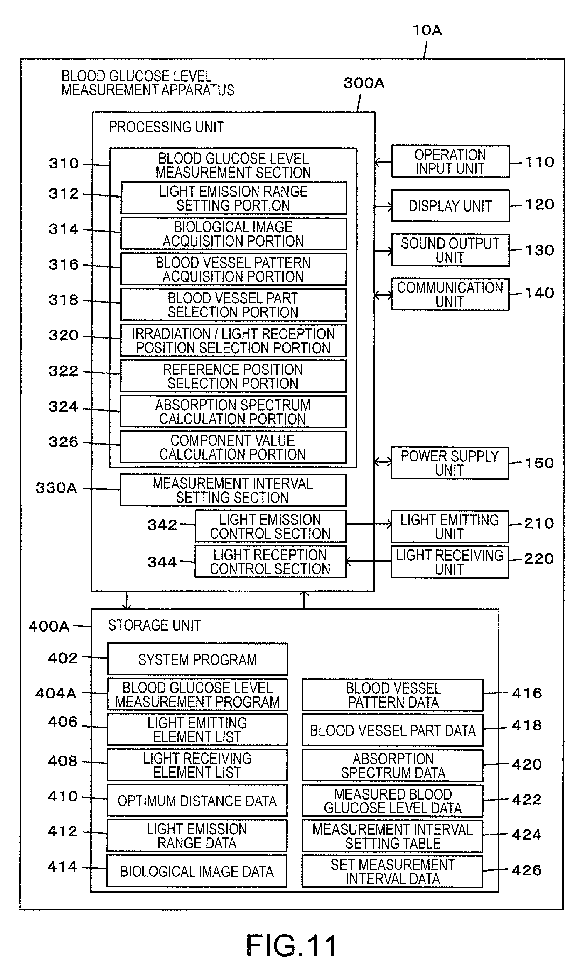

Fig. 11 is a functional configuration diagram of a blood glucose level measurement apparatus 10A in the first example. The blood glucose level measurement apparatus 10A functionally includes an operation input unit 110, a display unit 120, a sound output unit 130, a communication unit 140, a light emitting unit 210, a light receiving unit 220, a processing unit 300A, and a storage unit 400A.

The operation input unit 110 is an input device such as a button switch, a touch panel, or various sensors, and outputs an operation signal corresponding to a performed operation to the processing unit 300A. Various instruction inputs such as a measurement start instruction of a blood glucose level are performed via the operation input unit 110. In Fig. 1, the operation switch 18 or the touch panel 16 corresponds to the operation input unit 110.

The display unit 120 is a display device such as a liquid crystal display (LCD), and performs various displays based on display signals from the processing unit 300A. A measurement result and the like are displayed on the display unit 120. In Fig. 1, the touch panel 16 corresponds to the display unit 120.

The sound output unit 130 is a sound output device such as a speaker, and outputs various sounds based on sound signals from the processing unit 300A. The sound output unit 130 outputs alarm sounds of measurement start or measurement end of a blood glucose level, the occurrence of a low blood glucose level, and the like.

The communication unit 140 is a communication device such as a wireless communication device, a modem, a wired communication cable jack, or a control circuit, and performs communication with external devices through connection to a communication line. In Fig. 1, the communication device 20 corresponds to the communication unit 140.

The light emitting unit 210 includes a plurality of light emitting elements 53 which are arranged in the planar shape in a two-dimensional manner. The light emitting layer 52 of the sensor module 50 illustrated in Figs. 2A and 2B corresponds to the light emitting unit 210. An arrangement position of the light emitting unit 210 (specifically, position coordinates of each light emitting element 53 in the Xs-Ys orthogonal coordinate system) is stored as a light emitting element list 406.

The light receiving unit 220 includes a plurality of light receiving elements 59 which are arranged in the planar shape in a two-dimensional manner. The light receiving layer 58 of the sensor module 50 illustrated in Figs. 2A and 2B corresponds to the light receiving unit 220. An arrangement position of the light receiving unit 220 (specifically, position coordinates of each light receiving element 59 in the Xs-Ys orthogonal coordinate system) is stored as a light receiving element list 408.

The processing unit 300A, which is implemented by, for example, a microprocessor such as a CPU or a graphics processing unit (GPU), or an electronic part such as an application specific integrated circuit (ASIC) or an IC memory, performs various calculation processes on the basis of a predetermined program or data or an operation signal from the operation input unit 110, and controls an operation of the blood glucose level measurement apparatus 10A. In Fig. 1, the control board 30 corresponds to the processing unit 300A. In the first example, the processing unit 300A includes a blood glucose level measurement section 310, a measurement interval setting section 330A, a light emission control section 342, and a light reception control section 344.

The blood glucose level measurement section 310 includes a light emission range setting portion 312, a biological image acquisition portion 314, a blood vessel pattern acquisition portion 316, a blood vessel part selection portion 318, an irradiation/light reception position selection portion 320, a reference position selection portion 322, an absorption spectrum calculation portion 324, and a component value calculation portion 326, and measures a glucose concentration in blood of the user 2, that is, a blood glucose level.

The light emission range setting portion 312 sets the light emission range 60 of the light emitting elements 53 in the light emitting unit 210. In other words, in the light emission surface of the light emitting unit 210, the light emission range 60 which is a circular range with the predetermined radius R centering on an irradiation position (the measurement light emitting element 53a) in the previous measurement is set. The irradiation position (the measurement light emitting element 53a) in the previous measurement is included in blood vessel part data 418. In addition, the light emission range 60 set by the light emission range setting portion 312 is stored as light emission range data 412.

Here, in a case where there are a plurality of irradiation positions (the measurement light emitting elements 53a) in the previous measurement, a plurality of light emission ranges 60 which respectively correspond to the plurality of irradiation positions, or light emission ranges 60 which respectively correspond to one or a plurality of irradiation positions selected from among the plurality of irradiation positions may be set. In a case of an initial measurement, there is no irradiation position in the previous measurement, and thus a range (that is, the entire light emission surface of the light emitting unit 210) including all the light emitting elements 53 is set as the light emission range 60.

The biological image acquisition portion 314 acquires a biological image of the user 2. The acquisition of a biological image is performed by appropriately using a biological image capturing technique in the well-known vein authentication technique or the like. In other words, among the light emitting elements 53 of the light emitting unit 210, the light emitting elements 53 within the light emission range 60 set by the light emission range setting portion 312 simultaneously emit light, and all the light receiving elements 59 perform photometry (perform imaging). A luminance image based on a photometry result, that is, a biological image, is generated. The biological image acquired by the biological image acquisition portion 314 is stored as biological image data 414.

The blood vessel pattern acquisition portion 316 performs a predetermined image process on the biological image acquired by the biological image acquisition portion 314, so as to acquire a blood vessel pattern. Specifically, the acquisition is performed by appropriately using a technique for identifying a vein pattern from a biological image in the well-known vein authentication technique. For example, a binarization or a filtering process is performed on each pixel of the biological image through comparison with reference luminance. A pixel having luminance lower than the reference luminance indicates a blood vessel region, and a pixel having luminance equal to or higher than the reference luminance indicates a non-blood vessel region. The blood vessel pattern acquired by the blood vessel pattern acquisition portion 316 is stored as blood vessel pattern data 416.

The blood vessel part selection portion 318 selects the blood vessel part 6 satisfying a predetermined selection condition as a measurement target on the basis of the blood vessel pattern acquired by the blood vessel pattern acquisition portion 316. Here, the blood vessel part 6 may be selected alone or in a plurality as a measurement target. Each blood vessel part 6 selected as a measurement target is stored as blood vessel part data 418.

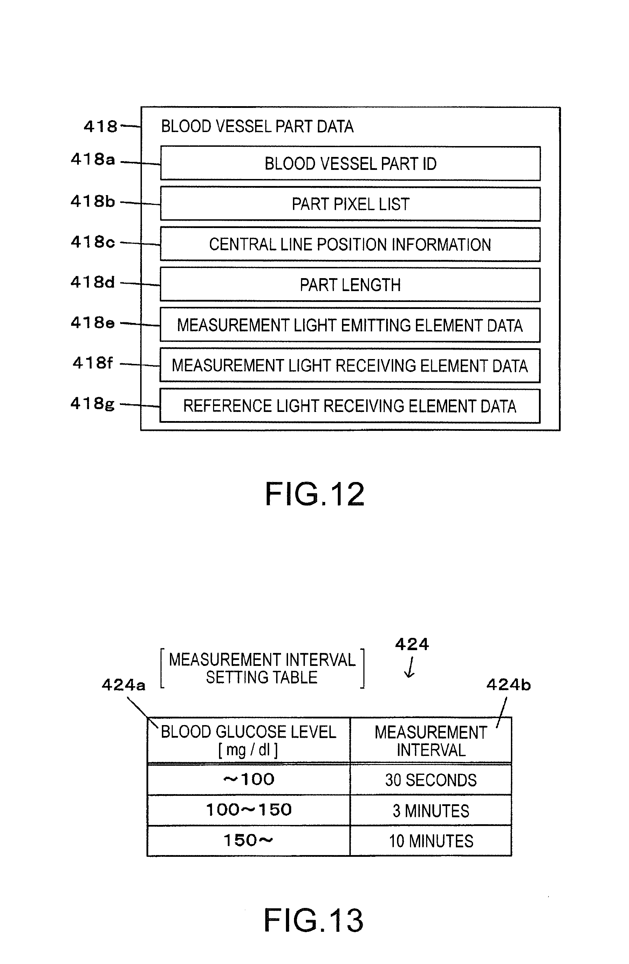

Fig. 12 is a diagram illustrating an example of a configuration of the blood vessel part data 418. The blood vessel part data 418 stores a blood vessel part ID 418a which is identification information of the corresponding blood vessel part, a part pixel list 418b, central line position information 418c, a part length 418d which is a length in the blood vessel length direction, measurement light emitting element data 418e, measurement light receiving element data 418f, and reference light receiving element data 418g. The part pixel list 418b is a list of pixels (that is, the light receiving elements 59) corresponding to the blood vessel part. The central line position information 418c is information on position coordinates of a central line (which is a center in the blood vessel width direction and is a line in the blood vessel length direction) of the blood vessel part in the Xs-Ys orthogonal coordinate system.

The irradiation/light reception position selection portion 320 selects an irradiation position (the measurement light emitting element 53a) and a measurement light reception position (the measurement light receiving element 59a) so that each measurement target blood vessel part 6 satisfies the first relative position condition. Specifically, the irradiation position and the measurement light reception position are set to two positions, with the blood vessel part 6 interposed therebetween, which have the optimum distance W as a gap therebetween in two directions perpendicular to the central line from a certain position on the central line of the blood vessel part 6 in the Xs-Ys orthogonal coordinate system (that is, in the skin surface). In addition, the light emitting element 53 at the irradiation position is used as the measurement light emitting element 53a, and the light receiving element 59 at the measurement light reception position is used as the measurement light receiving element 59a. The optimum distance W is stored as optimum distance data 410. As a method of selecting a certain position on the central line, an approximately central position of the blood vessel part 6 in the length direction is defined.

In a case where there are no positions (that is, positions which can be set as the irradiation position and the measurement light reception position) satisfying the first relative position condition for the selected certain position, it is similarly determined whether or not there are positions (that is, positions which can be set as the irradiation position and the measurement light reception position) satisfying the first relative position condition for a position far from the certain position along the central line by a predetermined unit distance. In a case where positions satisfying the first relative position conditions are not found yet, the process is repeatedly performed in the same manner, and the measurement irradiation position and the measurement light reception position are searched for and set.

The reference position selection portion 322 selects a reference light reception position (the reference light receiving element 59b) so as to satisfy the second relative position condition with respect to the irradiation position and the measurement light reception position set by the irradiation/light reception position selection portion 320. In addition, in a case where there is no position satisfying the second relative position condition, the irradiation position and the measurement light reception position are searched for and set again by the irradiation/light reception position selection portion 320.

The absorption spectrum calculation portion 324 generates an absorption spectrum for each measurement target blood vessel part 6. Specifically, the absorption spectrum is generated by calculating the transmittance T for each wavelength λ on the basis of a light reception result (light intensity) obtained from the measurement light receiving element 59a and the reference light receiving element 59b set for the blood vessel part 6. In addition, in a case where there are a plurality of measurement target blood vessel parts 6, respective absorption spectra of the plurality of measurement target blood vessel parts 6 are averaged, and thus an average absorption spectrum is calculated. The absorption spectrum calculated by the absorption spectrum calculation portion 324 is stored as absorption spectrum data 420.

The component value calculation portion 326 calculates a glucose concentration (that is, a blood glucose level) which is a concentration in blood of an aimed blood component on the basis of the absorption spectrum calculated by the absorption spectrum calculation portion 324. In the present embodiment, the absorption spectrum is obtained by using an analysis method such as a multiple regression analysis method, a main component regression analysis method, a PLS regression analysis method, or an independent component analysis method. In a case where there are a plurality of measurement target blood vessel parts 6, a blood glucose level is calculated on the basis of an average absorption spectrum which is obtained by averaging absorption spectra related to the respective blood vessel parts 6. The blood glucose level calculated by the component value calculation portion 326 is accumulated and stored as measured blood glucose level data 422 in correlation with the measurement time.

The measurement interval setting section 330A sets a measurement interval at which the blood glucose level measurement section 310 measures a blood glucose level. Specifically, a measurement interval is set according to a measurement interval setting table 424 on the basis of a blood glucose level measured by the blood glucose level measurement section 310. The measurement interval set by the measurement interval setting section 330A is stored as set measurement interval data 426.

Fig. 13 is a diagram illustrating an example of a data configuration of the measurement interval setting table 424. As illustrated in Fig. 13, the measurement interval setting table 424 stores a blood glucose level 424a and a measurement interval 424b in correlation with each other. In Fig. 13, as the blood glucose level 424a becomes higher, the measurement interval 424b is set to become longer.

The light emission control section 342 selectively controls light emission of each of the plurality of light emitting elements 53 of the light emitting unit 210. The light reception control section 344 acquires an amount of received light from each of the plurality of light receiving elements 59 of the light receiving unit 220.

The storage unit 400A, which is a storage device such as a ROM, a RAM, or a hard disk, stores a program, data, or the like for the processing unit 300A collectively controlling the blood glucose level measurement apparatus 10A, and is used as a work area of the processing unit 300A so as to temporarily store a result of calculation performed by the processing unit 300A or operation data from the operation input unit 110. In Fig. 1, the main memory or the measurement data memory mounted on the control board 30 corresponds to the storage unit 400A. The storage unit 400A stores a system program 402, a blood glucose level measurement program 404A, the light emitting element list 406, the light receiving element list 408, the optimum distance data 410, the light emission range data 412, the biological image data 414, the blood vessel pattern data 416, the blood vessel part data 418, the absorption spectrum data 420, the measured blood glucose level data 422, the measurement interval setting table 424, and the set measurement interval data 426.

### Flow of Process

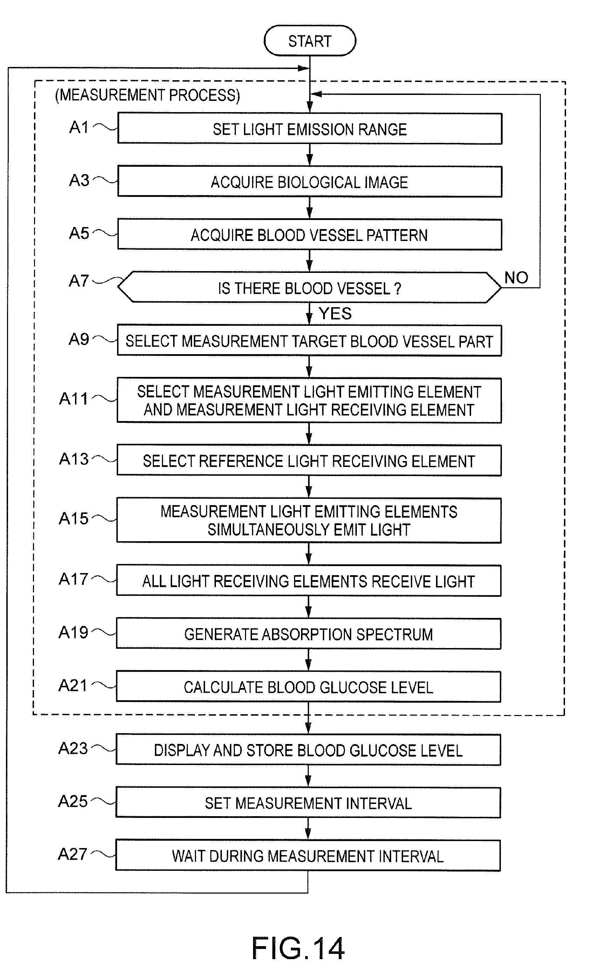

Fig. 14 is a flowchart illustrating a flow of a blood glucose level measurement process in the first example. This process is realized by the processing unit 300A performing a process according to the blood glucose level measurement program 404A.

In Fig. 14, first, the blood glucose level measurement section 310 performs a measurement process of measuring a blood glucose level of the user. In other words, the light emission range setting portion 312 sets the light emission range 60 of the sensor module 50 (step A1). That is, in a first measurement, the entire light emission surface (that is, a range including all the light emitting elements 53) of the sensor module 50 is set as the light emission range 60, and, in the second and subsequent measurements, a range centering on an irradiation position (the measurement light emitting element 53a) in the previous measurement is used as the light emission range 60.

Next, among the light emitting elements 53 of the sensor module 50, only the light emitting elements 53 in the set light emission range 60 are made to emit light, and thus the biological image acquisition portion 314 acquires a biological image of the user (step A3). Next, the blood vessel pattern acquisition portion 316 acquires a blood vessel pattern which is viewed from the skin surface on the basis of the obtained biological image (step A5). If a blood vessel pattern cannot be obtained (step A7: NO), the flow returns to step A1, and the light emission range 60 is reset. At this time, for example, the light emission range 60 is reset to be widened by increasing the radius R by a predetermined length ΔR.

If the blood vessel pattern is obtained (step A7: YES), the blood vessel part selection portion 318 selects the measurement target blood vessel part 6 satisfying a predetermined selection condition on the basis of the obtained blood vessel pattern (step A9). The irradiation/light reception position selection portion 320 selects an irradiation position (the measurement light emitting element 53a) and a measurement light reception position (the measurement light receiving element 59a) satisfying the first relative position condition for each measurement target blood vessel part 6 (step A11). Next, the reference position selection portion 322 selects a reference light reception position (the reference light receiving element 59b) satisfying the second relative position condition for each measurement target blood vessel part 6 (step A13).

Next, the measurement light emitting elements 53a set for the respective measurement target blood vessel parts 6 simultaneously emit light (step A15), and all the light receiving elements 59 receive light (perform imaging) (step A17). Next, the absorption spectrum calculation portion 324 generates an absorption spectrum for the blood vessel part 6 on the basis of a light reception result (light intensity) obtained from the respective measurement light receiving elements 59a and reference light receiving elements 59b set for each measurement target blood vessel part 6. In addition, in a case where there are a plurality of measurement target blood vessel parts 6, an absorption spectrum is calculated by averaging absorption spectra for the respective blood vessel parts 6 (step A19). Then, the component value calculation portion 326 calculates a glucose concentration in blood, that is, a blood glucose level on the basis of the absorption spectrum (step A21). The calculated blood glucose level is displayed on the display unit 120, and is accumulated and stored in correlation with the measurement time (step A23).

Next, the measurement interval setting section 330A sets a measurement interval to the next measurement according to the measured blood glucose level (step A25). A wait occurs while the set measurement interval elapses (step A27), and the flow returns to step A1, and the next measurement of a blood glucose level is performed in the same manner.

### Second Example

Next, a second example will be described. In the following description, the same constituent elements as in the above-described first example are given the same reference numerals, and detailed description thereof will be omitted.

### Overview

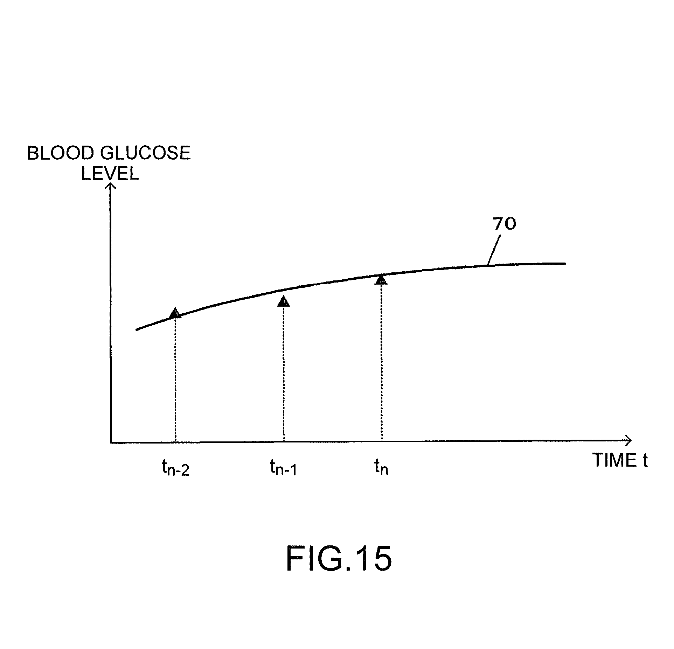

In the second example, a subsequent blood glucose level is predicted on the basis of a measurement result of a blood glucose level, and a measurement interval to the next measurement is set on the basis of a prediction result. Fig. 15 is a diagram for explaining prediction of a blood glucose level. In Fig. 15, a transverse axis expresses the time t, and a longitudinal axis expresses a blood glucose level. Fig. 15 is a diagram in which a plurality of measurement results (a blood glucose level for the measurement time t) in the past are plotted. On the basis of these measurement results, by using, for example, approximate calculation such as a least square method, a prediction curve 70 indicating a change (transition over time) in a blood glucose level after the previous measurement time tₙ is generated.

In addition, the prediction result of a blood glucose level is adjusted according to an action of the user 2. A blood glucose level changes due to a predetermined action of the user 2, such as a meal, insulin administration, or exercise. For this reason, the prediction result of a blood glucose level is adjusted according to a scheduled action of the user 2. In other words, the prediction curve 70 of a blood glucose level is adjusted according to the type of scheduled action after a scheduled action time.

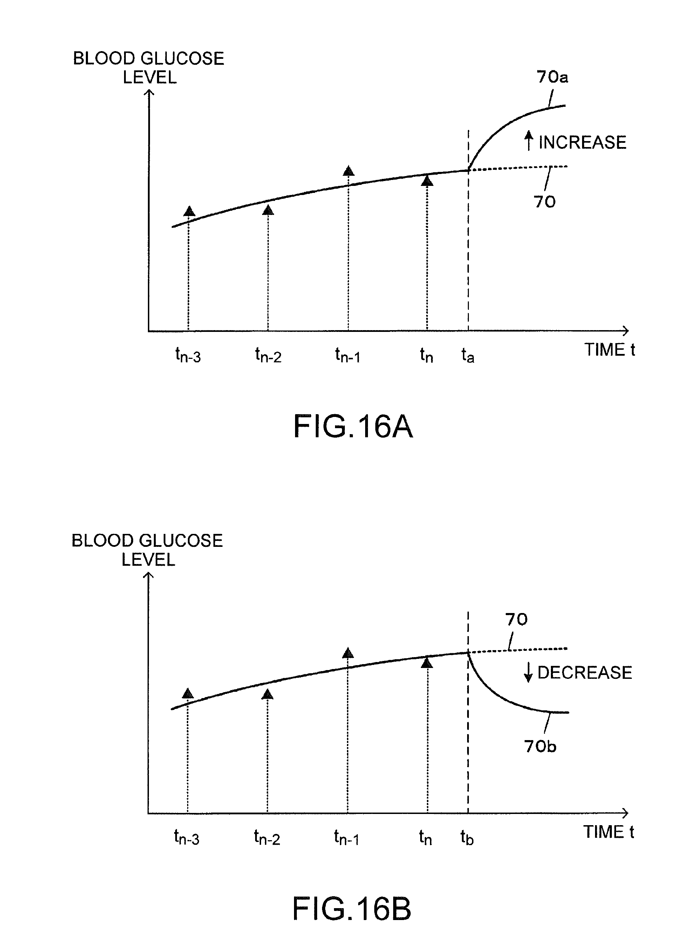

Figs. 16A and 16B are diagrams for explaining adjustment of a prediction result of a blood glucose level according to a scheduled action of the user. Fig. 16A illustrates a case where a meal is scheduled. A blood glucose level changes so as to increase due to the meal. Therefore, in this case, in the prediction curve 70 of a blood glucose level, the portion after the scheduled time tₐ of the action is changed (adjusted) so that a blood glucose level is increased.

Fig. 16B illustrates a case where exercise or insulin administration is scheduled. A blood glucose level changes so as to decrease due to the exercise or the insulin administration. Therefore, in this case, in the prediction curve 70 of a blood glucose level, the portion after the scheduled time t_{b} of the action is changed (adjusted) so that a blood glucose level is decreased.

If the blood glucose level is predicted, then, a low blood glucose prediction time which is a point of time at which the blood glucose level satisfies a predetermined low blood glucose condition is determined on the basis of the prediction result. The low blood glucose condition is a condition in which a blood glucose level is regarded to be in danger of low blood glucose, and is, specifically, that a blood glucose level is equal to or lower than a predetermined reference blood glucose level (for example, about 100 [mg/dl]).

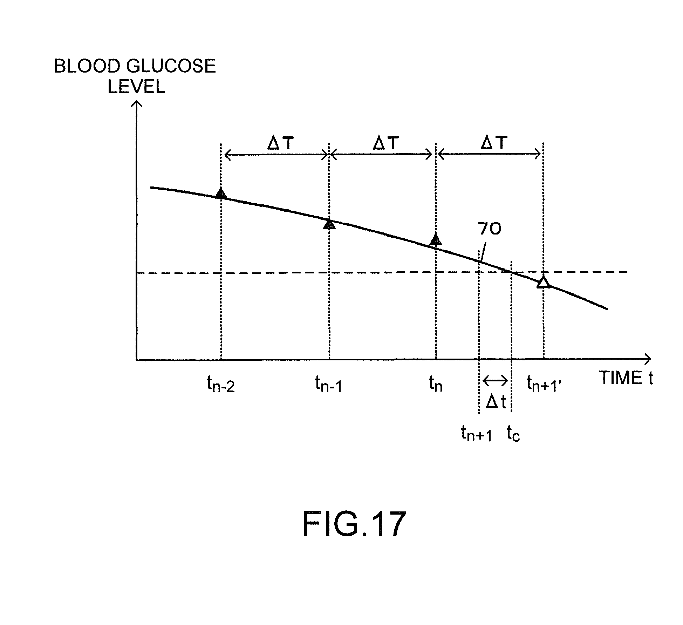

Fig. 17 is a diagram for explaining determination of the low blood glucose prediction time. In an example illustrated in Fig. 17, the prediction curve 70 of a blood glucose level changes so as to decrease, and thus the blood glucose level is equal to or lower than the reference blood glucose level at the time point t_{c}. This time point t_{c} is the low blood glucose prediction time. A measurement interval is set so that the next measurement is performed before the low blood glucose prediction time t_{c}. For example, it is assumed that a measurement interval is the time ΔT, and measurements are performed at the time point tₙ₋₂, the time point tₙ₋₁, and the time point tₙ. The next measurement is performed at the time point tₙ₊₁, when ΔT has elapsed from the time point tₙ, but the low blood glucose prediction time t_{c} comes between the time point tₙ and the time point t_{n+1'}. Therefore, the next measurement is performed at the next measurement time point tₙ₊₁ which is a predetermined time Δt before the low blood glucose prediction time t_{c}, and a measurement interval is set to a gap between the measurement time point tₙ and the next measurement time point tₙ₊₁.

### Functional Configuration

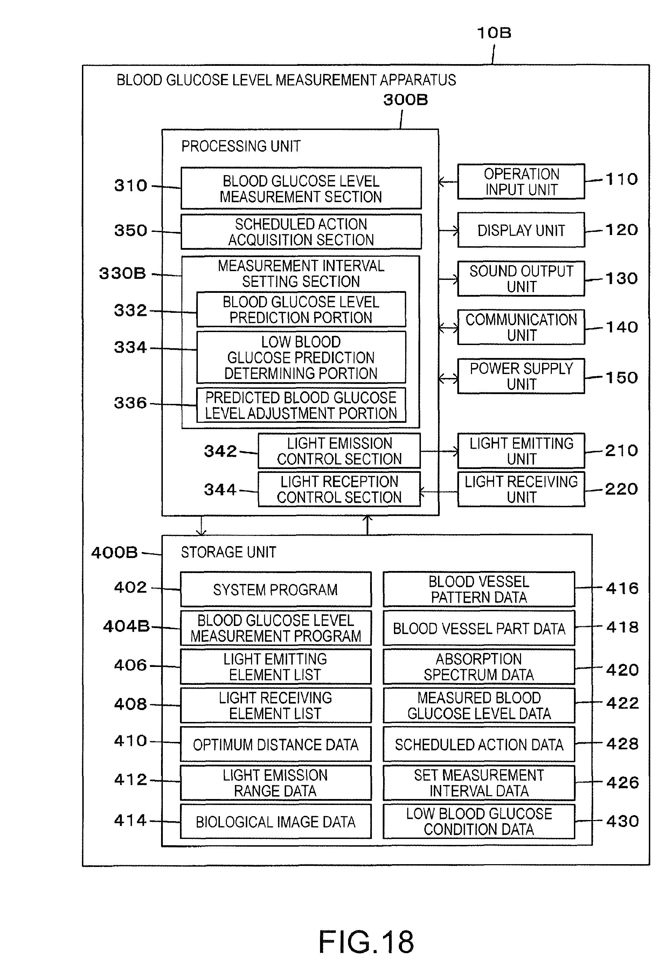

Fig. 18 is a block diagram illustrating a functional configuration of a blood glucose level measurement apparatus 10B in the second example. As illustrated in Fig. 18, in the blood glucose level measurement apparatus 10B, a processing unit 300B includes a blood glucose level measurement section 310, a scheduled action acquisition section 350, and a measurement interval setting section 330B.

The scheduled action acquisition section 350 acquires a predetermined action (scheduled action) which is scheduled by the user 2 and influences a blood glucose level, along with the action time. The predetermined action is, for example, a meal, insulin administration, exercise, or the like. As a method of acquiring the information, for example, the user may input the information via the operation input unit 110, or the information may be acquired from an external device via the communication unit 140.

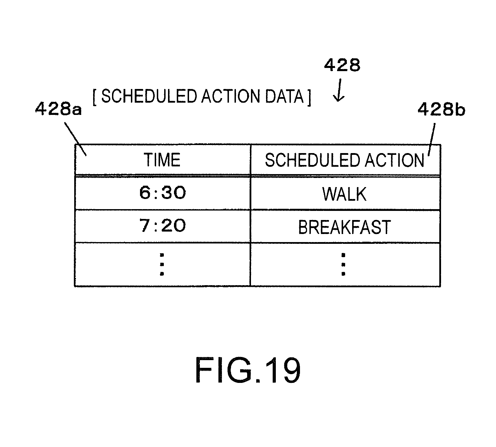

The scheduled action acquired by the scheduled action acquisition section 350 is stored as scheduled action data 428. Fig. 19 is a diagram illustrating an example of a configuration of the scheduled action data 428. As illustrated in Fig. 19, the scheduled action data 428 stores time 428a and a scheduled action 428b in correlation with each other.

The measurement interval setting section 330B includes a blood glucose level prediction portion 332, a low blood glucose prediction determining portion 334, and a predicted blood glucose level adjustment portion 336, and sets a measurement interval of a blood glucose level performed by the blood glucose level measurement section 310.

The blood glucose level prediction portion 332 predicts a future change in a blood glucose level on the basis of measurement results (the measured blood glucose level data 422) of a blood glucose level hitherto. In other words, for example, through predetermined approximate calculation based on the measurement results of a blood glucose level, a prediction curve 70 indicating a change in a blood glucose level after the previous measurement time tₙ is generated.

The predicted blood glucose level adjustment portion 336 adjusts the blood glucose level predicted by the blood glucose level prediction portion 332. In other words, it is determined whether or not an action of the user which influences a blood glucose level is scheduled within a predetermined time (for example, within an hour) from the present time on the basis of the scheduled action of the user (the scheduled action data 428) acquired by the scheduled action acquisition section 350. If there is a scheduled action, the prediction curve 70 is adjusted so that a blood glucose level is increased or decreased from the scheduled time depending on the type of corresponding action.

The low blood glucose prediction determining portion 334 determines a low blood glucose prediction time which is a point of time at which the blood glucose level satisfies a predetermined low blood glucose condition on the basis of the prediction result of a blood glucose level. Here, the low blood glucose condition is that "a blood glucose level is equal to or lower than a predetermined reference blood glucose level", and the reference blood glucose level is stored as low blood glucose condition data 430. In other words, the low blood glucose prediction determining portion 334 determines a point of time at which a blood glucose level given by the prediction curve 70 is equal to or lower than the predetermined reference blood glucose level, as the low blood glucose prediction time.

The measurement interval setting section 330B sets a measurement interval to the next measurement of a blood glucose level on the basis of the low blood glucose prediction time determined by the low blood glucose prediction determining portion 334. In other words, assuming that the next measurement time is temporarily defined with a predetermined value (for example, three minutes) as a measurement interval, in a case where it is determined that the next measurement time is after the low blood glucose prediction time, the measurement interval is changed and set (hereinafter, referred to as "special setting") so that the next measurement is performed before the low blood glucose prediction time. On the other hand, in a case where it is determined that the low blood glucose prediction time does not arrive before the temporarily defined measurement time, the predetermined value (for example, three minutes) is used and set as a measurement interval. In addition, in the present example, the special setting is performed once for a low blood glucose prediction time. In other words, after the special setting is performed once for a certain low blood glucose prediction time, the next measurement time after the specially set measurement time is returned to the predetermined value measurement interval. However, instead of being returned to the predetermined value, a short time interval (for example, 15 seconds) which is shorter than the predetermined value may be set as a measurement interval for a while (during a predetermined period of time), and then the next measurement time is returned to the predetermined value after the predetermined period of time has elapsed.

A storage unit 400B stores the system program 402, a blood glucose level measurement program 404B, the light emitting element list 406, the light receiving element list 408, the optimum distance data 410, the light emission range data 412, the biological image data 414, the blood vessel pattern data 416, the blood vessel part data 418, the absorption spectrum data 420, the measured blood glucose level data 422, the set measurement interval data 426, the scheduled action data 428, and the low blood glucose condition data 430.

### Flow of Process

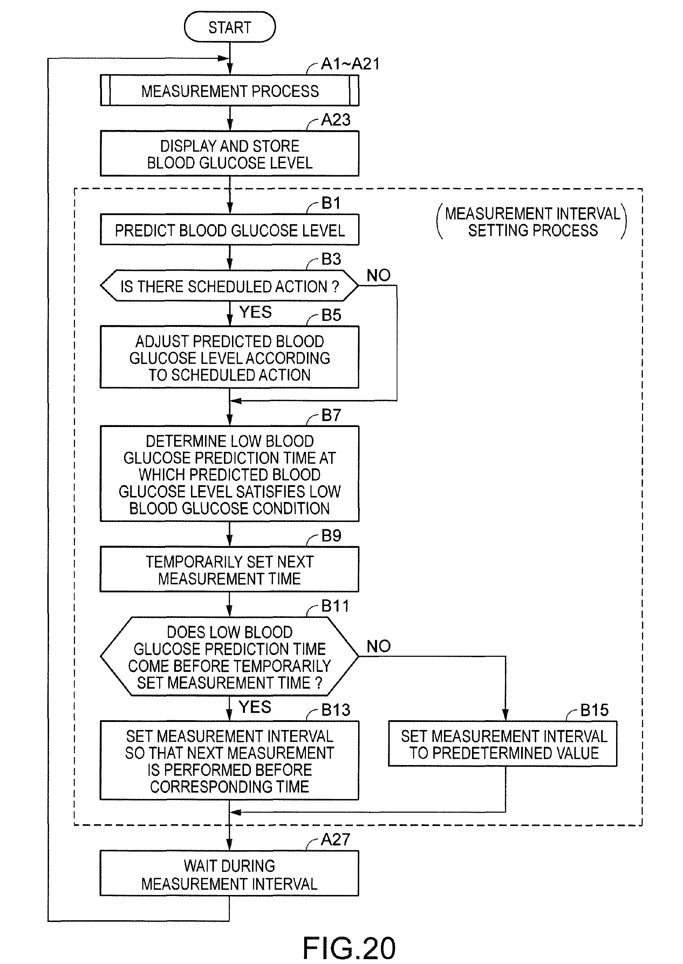

Fig. 20 is a flowchart illustrating a flow of a blood glucose level measurement process B in the second example. This process is realized by the processing unit 300B performing a process according to the blood glucose level measurement program 404B.

In Fig. 20, first, the blood glucose level measurement section 310 performs a blood glucose level measurement process so as to measure a blood glucose level of the user (steps A1 to A21). In addition, the measured blood glucose level is displayed on the display unit 120 and is stored in correlation with a measurement result (step A23).

Next, the measurement interval setting section 330B performs a measurement interval setting process so as to set a measurement interval to the next measurement. In other words, the blood glucose level prediction portion 332 predicts a blood glucose level on the basis of the measurement result of a blood glucose level (step B1). Next, the predicted blood glucose level adjustment portion 336 determines whether or not there is a scheduled action of the user, and adjusts the predicted blood glucose level depending on the type of scheduled action (step B5) if there is the scheduled action (step B3: YES).

Next, the low blood glucose prediction determining portion 334 determines a low blood glucose prediction time satisfying a predetermined low blood glucose condition on the basis of the prediction result of a blood glucose level (step B7). The next measurement time is calculated when a temporary measurement interval is set to a predetermined value (step B9). If it is determined that the temporarily defined next measurement time comes after the low blood glucose prediction time (that is, the low blood glucose prediction time comes between the present time and the temporarily defined next measurement time) (step B11: YES), the measurement interval setting section 330B sets a measurement interval to the next measurement so that the next measurement is performed before the low blood glucose prediction time (step B13). If there is no low blood glucose prediction time between the present time and the temporarily defined next measurement time (step B11: NO), the measurement interval setting section 330B sets a measurement interval to the predetermined value (step B15) . As mentioned above, if the measurement interval is set, a wait occurs by the set measurement interval (step A27), and then the flow returns to step A1 so that the next measurement of a blood glucose level is performed in the same manner.

### Operations and Effects

As mentioned above, according to the present embodiment, a measurement interval to the next measurement is dynamically set and changed according to a measurement result of a blood glucose level, and thus it is possible to rapidly detect a low blood glucose state and to reduce power consumption. In other words, in the first example, as a measured blood glucose level becomes lower, a measurement interval is set to become shorter. In addition, in the second example, a subsequent blood glucose level is predicted on the basis of a measured value of a blood glucose level, and a measurement interval is set according to the predicted blood glucose level. Consequently, the occurrence of a low blood glucose state is predicted, and an appropriate measurement interval can be set so that the low blood glucose state can be rapidly detected. In addition, a predicted blood glucose level is adjusted according to a scheduled action of a user, and thus it is possible to predict a more appropriate blood glucose level.

### Modification Examples

An applicable embodiment of the invention is not limited to the above-described embodiment and may include modifications within the scope not departing from the spirit of the invention as appropriate.

In the first example, a measured blood glucose level may be adjusted according to a scheduled action of the user, and a measurement interval is set according to the adjusted blood glucose level. Specifically, a measurement interval to the next measurement is set according to the measurement interval setting table 424 on the basis of the measured blood glucose level. Next, by referring to the scheduled action data 428, it is determined whether or not there is a scheduled action of the user 2 (scheduled action time comes) before the next measurement time which is obtained from the set measurement interval. If there is the scheduled action, the measured blood glucose level is adjusted by using a correction amount corresponding to the type of corresponding action, and a measurement interval is set again according to the measurement interval setting table 424 on the basis of the adjusted blood glucose level.

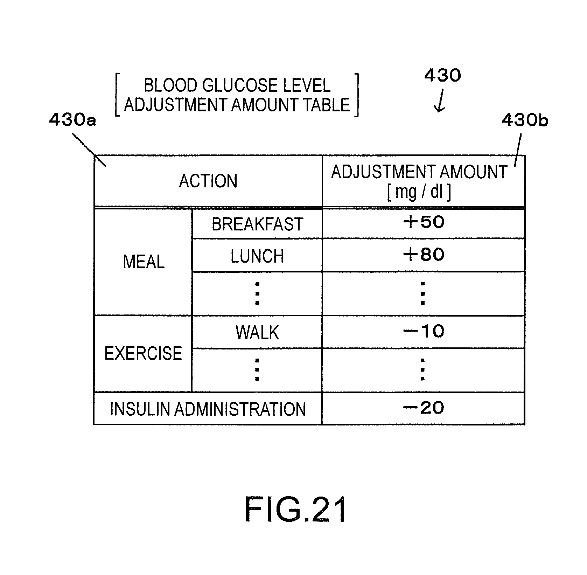

Here, an adjustment amount of a blood glucose level corresponding to the type of action is defined as illustrated in an example of Fig. 21. Fig. 21 is a diagram illustrating an example of a data configuration of a blood glucose level adjustment amount table 430. As illustrated in Fig. 21, the blood glucose level adjustment amount table 430 stores each type of action 430a in correlation with an adjustment amount 430b of a blood glucose level. A blood glucose level increases due to a meal, and decreases due to exercise or insulin administration. For this reason, in a case where a meal is scheduled until the next measurement, the measured blood glucose level is adjusted to increase, and in a case where exercise or insulin administration is scheduled, the measured blood glucose level is adjusted to decrease.

## Claims

1. A blood glucose level measurement apparatus comprising:
a measurement unit that measures a blood glucose level of a user; and
a measurement interval setting unit that sets a measurement interval performed by the measurement unit on the basis of a measurement result from the measurement unit.

2. The blood glucose level measurement apparatus according to claim 1, wherein the measurement interval setting unit sets a first measurement interval in a case where the measurement result is in a first range, and sets a second measurement interval shorter than the first measurement interval in a case where the measurement result is in a second range shorter than the first range.

3. The blood glucose level measurement apparatus according to claim 1 or claim 2, further comprising:
a storage unit that stores the measurement result,
wherein the measurement interval setting unit includes a prediction portion that predicts a blood glucose level by using a measurement result stored in the storage unit, and sets the measurement interval by using a prediction result.

4. The blood glucose level measurement apparatus according to any one of claims 1-3, wherein the measurement interval setting unit includes a determination portion that determines a point of time at which the prediction result satisfies a predetermined condition, and sets the measurement interval so that the measurement unit performs the next measurement at the point of time.

5. The blood glucose level measurement apparatus according to any one of claims 1-4, further comprising:
an acquisition unit that acquires a scheduled action of the user,
wherein the measurement interval setting unit includes a prediction result adjustment portion that adjusts the prediction result on the basis of the scheduled action.

6. The blood glucose level measurement apparatus according to claim 5, wherein the prediction result adjustment portion adjusts and reduces a predicted value of a blood glucose level included in the prediction result in a case where the scheduled action is an action related to exercise or insulin administration.

7. The blood glucose level measurement apparatus according to claim 5 or claim 6, wherein the prediction result adjustment portion adjusts and increases a predicted value of a blood glucose level included in the prediction result in a case where the scheduled action is an action related to a meal.

8. A blood glucose level measurement method comprising:
performing a measurement of a blood glucose level of a user; and
setting an interval of the measurement on the basis of a result of the measurement.
